# EUROPEAN PATENT SPECIFICATION

(11) **EP 2 181 641 B1**
(45) Date of publication and mention of the grant of the patent: **16.01.2013**
(21) Application number: 08790537.8
(22) Date of filing: 29.08.2008
(51) Int. Cl.: A61B 1/00, A61B 1/04, A61B 5/07, H03L 7/099, H04L 27/12

(54) **CAPSULE TYPE MEDICAL DEVICE**
MEDIZINISCHE KAPSELVORRICHTUNG
DISPOSITIF MEDICAL DE TYPE CAPSULAIRE

(30) Priority: 30.08.2007 JP 2007224767; 30.08.2007 JP 2007224771
(43) Date of publication of application: 05.05.2010
(73) Proprietor: Olympus Medical Systems Corp., Tokyo 151-0072 (JP)
(72) Inventor: HOMAN, Masatoshi, Tokyo 151-0072 (JP)
(74) Representative: von Hellfeld, Axel
(86) International application number: PCT/JP2008/002383
(87) International publication number: WO 2009/028214

(56) References cited:
- EP-A1- 1 702 555
- JP-A- 10 022 842
- JP-A- 2001 224 553
- JP-A- 2004 072 307
- JP-A- 2004 072 307
- JP-A- 2006 280 940
- US-A1- 2004 236 181
- US-A1- 2006 241 422

## Description

### Technical Field

The present invention relates to a capsule type medical device, which takes the shape of a capsule, that is introduced into the inside of a living body in order to observe the inside of the living body, and that wirelessly transmits image data captured inside the living body to a receiving device outside the living body, and more particularly, to a technique for enabling image data to be stably transmitted even while an image is being captured.

### Background Art

Conventionally, various types of capsule type medical devices, such as a capsule type endoscope or the like, which take the shape of a small capsule in order to observe the inside of a living body, especially the inside of an organ of a digestive system of a patient, move sequentially inside the living body by being introduced from the mouth of the patient, and wirelessly transmit image data of the inside of the living body, which is captured during the moving, to a receiving device outside the living body are proposed.

Such capsule type medical devices mainly include an image capturing device such as a CCD camera, etc., which is intended to capture an image, and a transmitting device for wirelessly transmitting image data captured by the image capturing device to a receiving device outside the living body. In these capsule type medical devices, the image capturing device and the transmitting device are operated by using a button battery such as a 1.5-V silver oxide battery or the like as a power supply.
JP 2001-224553 discloses a capsule endoscope comprising a battery, an image sensor section, a video generating section, LEDs, a modulation part and a transmitting section. The capsule endoscope operates by alternatively switching ON the LEDs, the modulation part and the transmitting section during a transmitting cycle; and the image sensor section and the video generating section during a lighting sub-division cycle. The switching ON/OFF occurrences are triggered through the following devices: a 1st oscillator; a timing generator, inputted by the output of the 1st oscillator; and an inverter inverting the signal outputted by the timing generator.
JP 2004-072307 discloses a digital camera.

### Disclosure of Invention

However, the above described capsule type medical devices have a problem in that since the power supply for operating the image capturing device and the transmitting device is a small button battery, an output impedance increases, and a current consumed by the image capturing device fluctuates if the operation mode of the image capturing device is switched while the transmitting device is operating, leading to an occurrence of a ripple in a power supply line. For example, if the image capturing device starts to capture an image while the transmitting device is transmitting image data, a fault can possibly occur in the transmission of the image due to a drop in a voltage supplied to the transmitting device.
Additionally, an oscillation frequency of the transmitting device fluctuates due to a power supply ripple occurring in the image capturing device when a single type oscillator such as a Colpitts type oscillator is adopted as an oscillation circuit of the transmitting device. The fluctuations affect a wireless communication made by the transmitting device.
The present invention was developed in light of the above described problems, and an object thereof is to provide a capsule type medical device that can stably transmit image data even if the operation mode of the image capturing device is switched while the transmitting device is operating.
The present invention adopts the configuration defined in claim 1 in order to solve the above described problems.
Namely, a capsule type medical device in one aspect of the present invention is a capsule type medical device which is introduced into the inside of a living body in order to observe the inside of the living body. This capsule type medical device includes image capturing means for capturing an image of the inside of the living body, wireless communicating means for transmitting image data of the image captured by the image capturing means to the outside of the living body through a wireless communication, first voltage regulator means for regulating a voltage output from power supply means and for supplying the voltage to the image capturing means, and second voltage regulator means for regulating the voltage output from the power supply means and for supplying the voltage to the wireless communicating means, wherein an oscillating unit included in the wireless communicating means has a differential configuration.
The voltage value of the voltage supplied by the second regulator means is lower than that of the voltage supplied by the first regulator means in the capsule type medical device according to the present invention.
Furthermore, it is desirable that the oscillating unit is of a base-resonant scheme in the capsule type medical device according to the present invention.
Still further, it is desirable that the capsule type medical device according to the present invention includes a PLL (Phase Locked Loop), and also has a function of FSK (Frequency Shift Keying) modulation.
Still further, it is desirable that the oscillating unit is of a collector-resonant scheme in the capsule type medical device according to the present invention.
Still further, it is desirable that an oscillation inductor arranged between collector terminals of the oscillating unit also serves as an electromagnetic generation antenna included in the wireless communication device in the capsule type medical device according to the present invention.

### Brief Description of Drawings

Fig. 1 is a block diagram showing a capsule type medical device according to a first embodiment of the present invention;
Fig. 2 is a block diagram showing wireless communicating means in the first embodiment according to the present invention;
Fig. 3 is a circuit diagram showing an oscillating unit in the first embodiment according to the present invention;
Fig. 4 is a block diagram showing a capsule type medical device according to an example useful for understanding the present invention;
Fig. 5 is a block diagram showing wireless communicating means in the example of

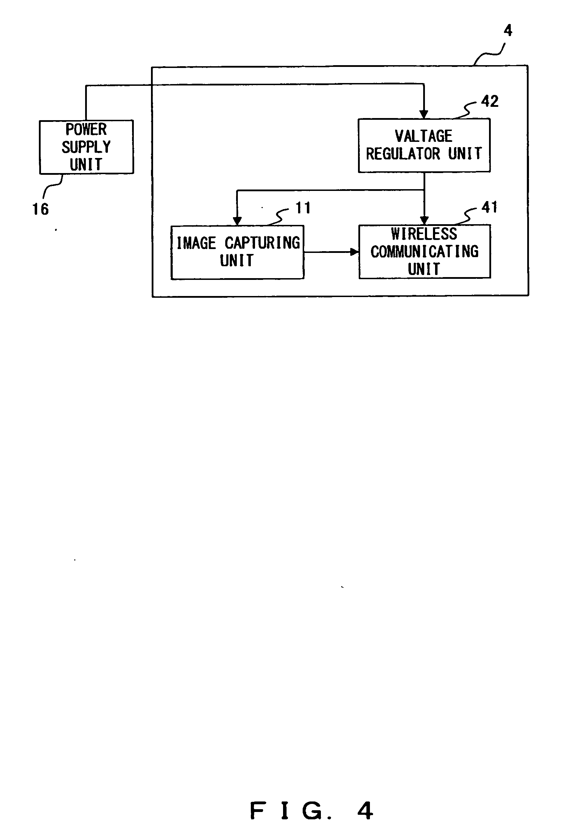

Fig. 4; and

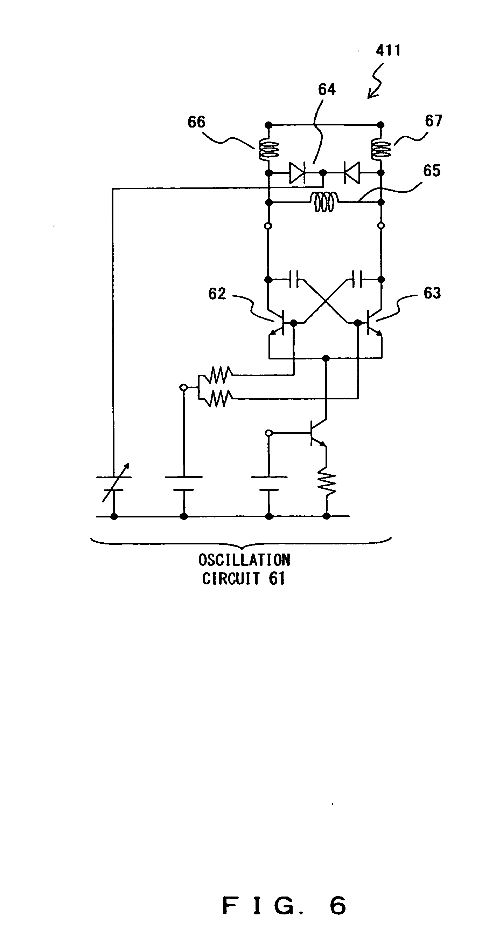

Fig. 6 is a circuit diagram showing an oscillating unit in the example of Fig. 4.

### Best Mode of Carrying Out the Invention

Embodiments according to the present invention are described below with reference to the drawings.
A first embodiment according to the present invention is described first.

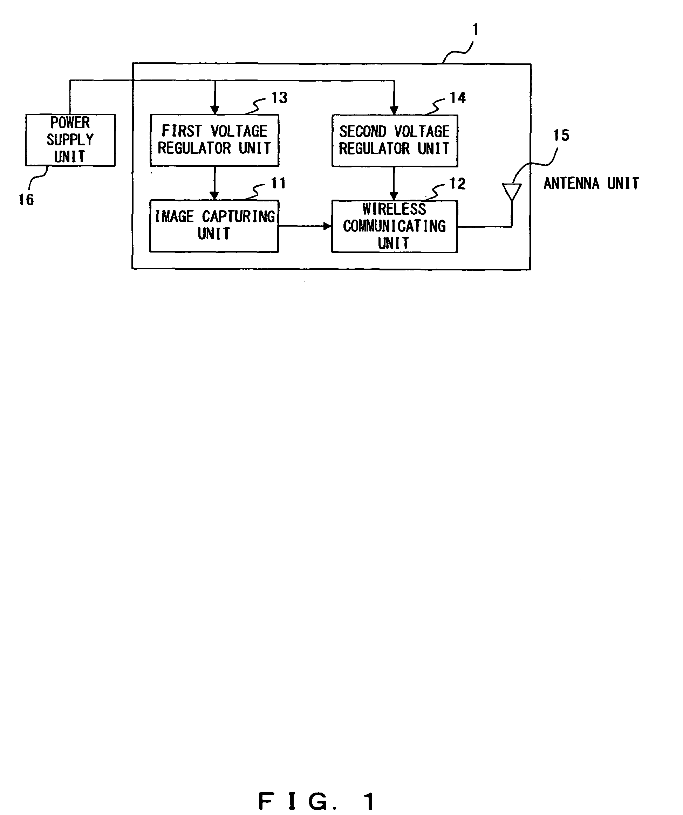

Fig. 1 is a block diagram showing a capsule type medical device according to the first embodiment of the present invention.
In Fig. 1, the capsule type medical device 1 is a capsule type endoscope or the like which takes the shape of a small capsule in order to observe the inside of a living body, especially the inside of an organ of a digestive system of a patient, sequentially moves inside the living body by being introduced from the mouth of the patient, and wirelessly transmits the image data of the inside of the living body, which is captured during the moving, to a receiving device outside the living body. The capsule type medical device 1 includes image capturing means 11 such as a CCD camera, etc., wireless communicating means 12, first voltage regulator means 13, second voltage regulator means 14, and antenna means 15.

The image capturing means 11 captures an image of the inside of the living body while the capsule type medical device 1 is moving and staying inside the living body.
The wireless communicating means 12 transmits the image data of the image captured by the image capturing means 11 to the receiving device outside the living body through a wireless communication via the antenna means 15.

The first voltage regulator means 13 regulates a voltage output from power supply means 16 such as a button battery, etc. , and supplies the voltage to the image capturing means 11.
The second voltage regulator means 14 regulates the voltage output from the power supply means 16, and supplies the voltage to the wireless communicating means 12.

Additionally, the voltage regulated by the second voltage regulator means 14 is set to a lower voltage than the first voltage regulator means 13 so that a voltage drop in the power supply means 16 such as a button battery, etc., exerts little influence on the image capturing device 11.

As described above, the voltage output from the same power supply means 16 is regulated by the first voltage regulator means 13 for the image capturing means 11, and is also regulated by the second voltage regulator means 14 for the wireless communicating means 12. Therefore, a stable voltage can be supplied to the wireless communicating means 12 even if the operation mode of the image capturing means 11 is switched while the wireless communicating means 12 is operating.

The second voltage regulator means 14 may be configured on the same IC as the wireless communicating means 12.

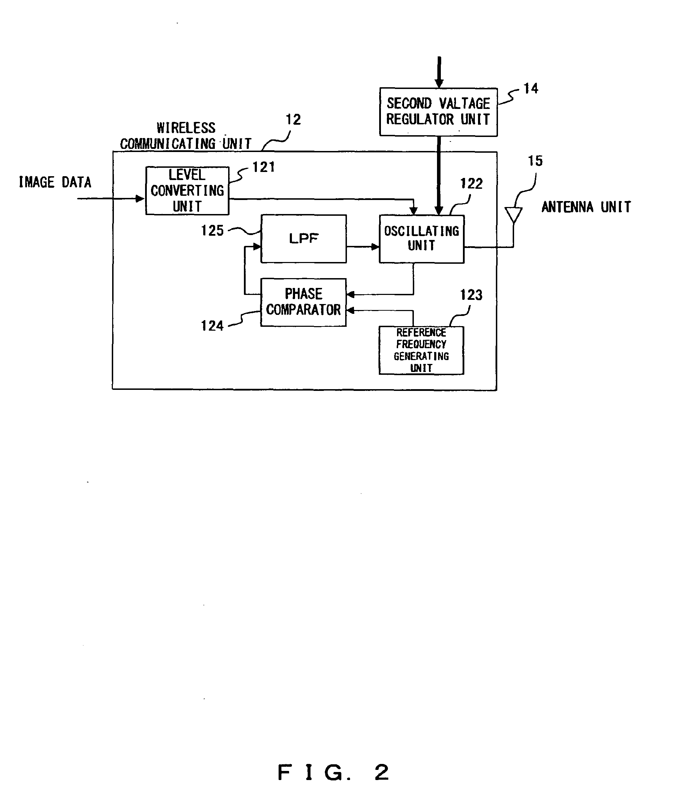

Fig. 2 is a block diagram showing the wireless communicating means in the first embodiment according to the present invention.

In Fig. 2, the wireless communicating means 12 includes a level converting unit 121, an oscillating unit 122, a reference frequency generating unit 123, a phase comparator 124, and a low-pass filter (LPF) 125.

The level converting unit 121 converts the voltage level of image data captured by the image capturing device 11, and inputs the voltage level to the oscillating unit 122.
The oscillating unit 122 oscillates and outputs a signal at a frequency based on the voltage level of the image data after being converted, which is input from the level converting unit 121, and the voltage level of the signal output from the LPF 125 at the voltage regulated by the second voltage regulator means 14. This oscillating unit 122 has a PLL (Phase Locked Loop) modulation circuit not shown, and also serves as an FSK (Frequency Shift Keying) modulator. Moreover, the frequency component of the input image data is set to a frequency higher than the cutoff frequency of the LPF 125.

The reference frequency generating unit 123 generates a reference frequency.
The phase comparator 124 makes a comparison between the phase of the signal output from the oscillating unit 122 and that of the reference frequency generated by the reference frequency generating unit 123, and outputs to the LPF 125 a signal according to a phase difference based on the result of the comparison.

The LPF 125 passes a low-frequency component of the input signal, and outputs the signal to the oscillating unit 122.

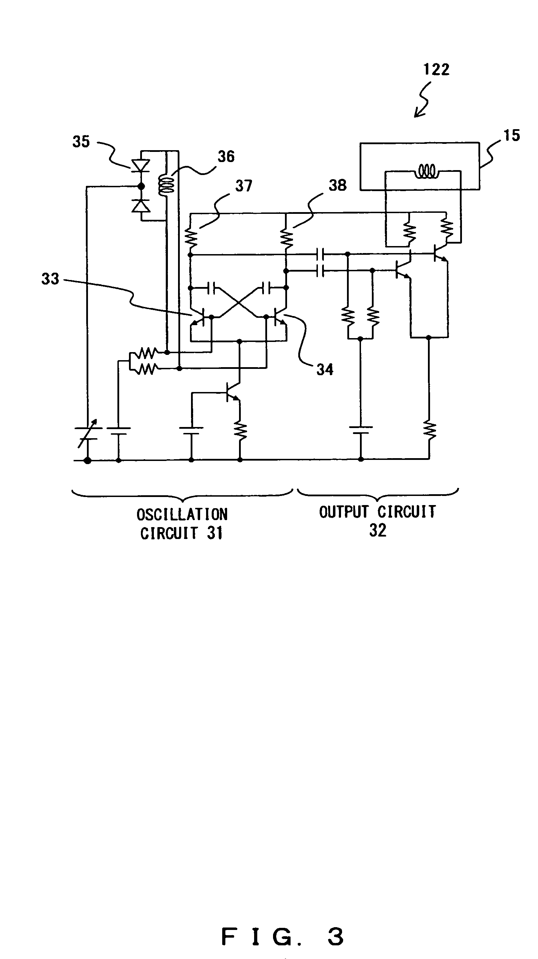

Fig. 3 is a circuit diagram showing the oscillating unit in the first embodiment according to the present invention.

In Fig. 3, the oscillating unit 122 is composed of an oscillation circuit 31 and an output circuit 32. A signal oscillated and output by the oscillation circuit 31 is output as radio waves via the output circuit 32 and the antenna means 15. The antenna means 15 is preferably a loop antenna or a dipole antenna, which is a balance type antenna, so that the signal can be efficiently output as radio waves from the oscillation circuit 31 having a differential configuration.

In the oscillation circuit 31, the base and the collector of one of transistors 33 and 34 are respectively cross-connected to the collector and the base of the other, and an output signal is output from the ground point of the two transistors 33 and 34. The cross-connections of the collectors and the bases of the two transistors 33 and 34 oscillate at the resonant frequency of an LC parallel resonant circuit. If an oscillation is made with a circuit having such a differential configuration, the influence of a ripple is reduced. Moreover, the oscillation circuit 31 having this differential configuration is an oscillation circuit of a base-resonant type.

Unlike a capsule type medical device 4 according to a second embodiment to be described later, a varicap diode 35 and a coil 36 are connected on the side of the base in the oscillation circuit 31 included in the capsule type medical device 1. Moreover, not coils instead but resistors 37 and 38 are connected for a pull-up in the power supply.

As described above, the capsule type medical device 1 according to the first embodiment of the present invention can improve the resistance to a power supply ripple by implementing the oscillation circuit 31 as a differential configuration. Additionally, by providing, on a power supply line for supplying the voltage to the oscillation circuit 31 included in the wireless device 12, a dedicated second voltage regulator 14 that is different from the first voltage regulator 13, which regulates the voltage supplied to the image capturing device 11, a power supply ripple that occurs in the image capturing device 11 can be removed as much as possible.
Furthermore, by combining these two configurations, in accordance with the capsule type medical device 1 being provided with the first voltage regulator means 13 for stabilizing the voltage supplied to the image capturing means 11 and the second voltage regulator means 14 for stabilizing the voltage supplied to the wireless communicating means 12, and the oscillating unit 122 being implemented as a differential configuration, the degree of stability of the frequency of the wireless communicating means 12 can be improved, and also the quality of the wireless communication can be improved. Accordingly, the capsule type medical device 1 according to the present invention can stably transmit image data even if the operation mode of the image capturing means 11 is switched while the wireless communicating means 12 is operating.
An example useful for understanding the present invention is described next.
Fig. 4 is a block diagram showing a capsule type medical device according to the present example.
In Fig. 4, similarly to the capsule type medical device 1 according to the first embodiment described with reference to Fig. 1, the capsule type medical device 4 is a capsule type endoscope, etc., which takes the shape of a small capsule in order to observe the inside of a living body, especially the inside of an organ of a digestive system of a patient, sequentially moves inside the living body by being introduced from the mouth of the patient, and wirelessly transmits the image data of the inside of the living body, which is captured during the moving, to a receiving device outside the living body. The capsule type medical device 4 includes the image capturing means 11 such as a CCD camera, etc., wireless communicating means 41, and voltage regulator means 42.
The image capturing means 11 captures an image of the inside of the living body while the capsule type medical device 4 is moving and staying inside the living body. The wireless communicating means 41 transmits the image data of the image captured by the image capturing means 11 to the receiving device outside the living body through a wireless communication via incorporated antenna means 15 of a differential type.
The voltage regulator means 42 regulates a voltage output from the power supply means 16 such as a button battery, etc., and supplies the voltage to both the image capturing means 11 and the wireless communicating means 41.

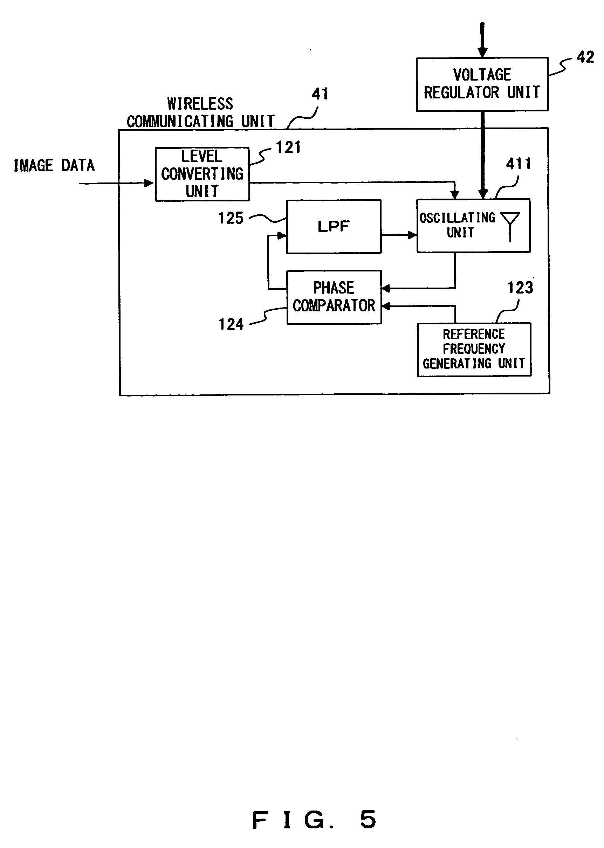

Fig. 5 is a block diagram showing the wireless communicating means in the present example.
In Fig. 5, the wireless communicating means 12 includes the level converting unit 121, an oscillating unit 411, the reference frequency generating unit 123, the phase comparator 124, and the low-pass filter (LPF) 125.
The level converting unit 121 converts the voltage level of image data captured by the image capturing device 11, and inputs the voltage level to the oscillating unit 411.
The oscillating unit 411 oscillates and outputs a signal based on the voltage level of the image data after being converted, the signal being input from the level converting unit 121, and the voltage level of the signal output from the LPF 125 at the voltage regulated by the voltage regulator means 42. This oscillating unit 411 incorporates antenna means of a balance type, includes a PLL modulation circuit not shown, and also serves as an FSK modulator similar to the oscillating unit 122 in the first embodiment described with reference to Fig. 2. Moreover, the frequency component of image data is set to a frequency higher than the cutoff frequency of the LPF 125.
The reference frequency generating unit 123 generates a reference frequency.
The phase comparator 124 makes a comparison between the phase of the signal output from the oscillating unit 411 and that of the reference frequency generated by the reference frequency generating unit 123, and outputs to the LPF 125 a signal according to a phase difference based on the result of the comparison.
The LPF 125 passes a low-frequency component of the input signal, and outputs the signal to the oscillating unit 411.
Fig. 6 is a circuit diagram showing the oscillating unit in the present example.
In Fig. 6, the oscillating unit 411 is composed of an oscillation circuit 61, and an oscillation inductor arranged between collector terminals is made to also serve as antenna means 65 for generating an electromagnetic field. A signal oscillated and output by the oscillation circuit 61 is output as radio waves via the antenna means 65. The shape of the antenna means 65 is preferably a loop or a spiral having an outer diameter of 3 mm or longer, which can form an inductor.
In the oscillation circuit 61, the base and the collector of one of transistors 62 and 63 are respectively cross-connected to the collector and the base of the other, and an output signal is output from the ground point of the two transistors 62 and 63. The cross-connections of the collectors and the bases of the two transistors 62 and 63 oscillate at the resonant frequency of an LC parallel resonant circuit. If an oscillation is made with a circuit having such a differential configuration, the influence of a ripple is reduced. Moreover, the oscillation circuit 61 having this differential configuration is an oscillation circuit of a collector-resonant type.
Unlike the above described capsule type medical device 1 according to the first embodiment, a varicap diode 64 and a coil as the antenna means 65 are connected on the side of the collector in order to make the coil also serve as the externally extending antenna means 65 in the oscillation circuit 61 included in the capsule type medical device 4. Moreover, not resistors but instead coils 66 and 67 are connected for a pull-up in the power supply.
As described above, the capsule type medical device 4 according to the present example can improve the resistance to a power supply ripple by implementing the oscillation circuit 61 as a differential configuration.
Additionally, by implementing the oscillation circuit 61 and the antenna means 65 as a collector-resonant type, the coil for resonance can be made to also serve as that for the antenna. This eliminates the need for the output circuit, leading to reductions in the power consumption and the size of the capsule type medical device 4.
As described above, according to the present invention, image data can be stably transmitted even if the operation mode of the image capturing device is switched while the transmitting device is operating, and power consumed by the operations of the transmitting device can be reduced.
The embodiments according to the present invention and an example useful for understanding the invention have been described up to this point. However, capsule type medical devices according to the present invention are not limited to the above described embodiments as far as their functions are carried out, and can take various configurations or shapes within a scope that does not depart from the gist of the present invention.
For example, the capsule type medical device 4 according to a second embodiment is configured so that the voltage regulator 42 regulates only the voltage supplied to the wireless communicating means 41, and a voltage regulator like the first voltage regulator 13 in the first embodiment, which regulates the voltage supplied to the image capturing means 11, is provided separately from the voltage regulator 42.

## Claims

1. A capsule type medical device to be introduced into an inside of a living body in order to observe the inside of the living body, comprising:
image capturing means (11) for capturing an image of the inside of the living body;
wireless communicating means (12) for transmitting image data of the image captured by the image capturing means (11) to an outside of the living body through a wireless communication;
first voltage regulator means (13) for regulating a voltage output from power supply means (16), and for supplying the voltage to the image capturing means (11); and
second voltage regulator means (14) for regulating the voltage output from the power supply means (16), and for supplying the voltage that is stable to the wireless communicating means (12), wherein
a value of the voltage supplied by the second voltage regulator means (14) is smaller than a value of the voltage supplied by the first voltage regulator means (13), and
an oscillating unit (122) comprised by the wireless communicating means (12) has a differential configuration.

2. The capsule type medical device according to claim 1, wherein
the oscillating unit (122) is of a base-resonant scheme.

3. The capsule type medical device according to claim 1, wherein
the oscillating unit has a PLL (Phase Locked Loop), and also serves an FSK, Frequency Shift Keying, modulation function.

4. The capsule type medical device according to claim 1, wherein
the oscillating unit (411) is of a collector-resonant scheme.

5. The capsule type medical device according to claim 4, wherein
an oscillation inductor (65) arranged between collector terminals of the oscillating unit (411) is made to also serve as an antenna for generating an electromagnetic field, and is comprised by the wireless communicating device (41).

## Patentansprüche

1. Medizinische Vorrichtung eines Kapseltyps zum Einfügen in das Innere eines lebenden Körpers, um das Innere des lebenden Körpers zu beobachten, aufweisend:
Bilderfassungsmittel (11) zum Erfassen eines Bildes des Inneren des lebenden Körpers;
drahtlose Kommunikationsmittel (12) zum Senden von Bilddaten des Bildes, welches durch die Bilderfassungsmittel (11) erfasst wurde, über drahtlose Kommunikation außerhalb des lebenden Körpers;
erste Spannungssteuerungsmittel (13) zum Steuern einer Spannungsausgabe von Energiezuführungsmitteln (16) und zum Zuführen der Spannung zu den Bilderfassungsmitteln (11); und
zweite Spannungssteuerungsmittel (14) zum Steuern der Spannungsausgabe der Energiezuführungsmittel (16) und zum Zuführen der Spannung, die stabil ist, zu den drahtlosen Kommunikationsmitteln (12), wobei
ein durch die zweiten Spannungssteuerungsmittel (14) zugeführter Spannungswert kleiner als ein durch die ersten Spannungssteuerungsmittel (13) zugeführter Spannungswert ist, und
eine Oszillationseinheit (122), die in den drahtlosen Kommunikationsmitteln (12) enthalten ist, eine differentielle Konfiguration aufweist.

2. Medizinische Vorrichtung eines Kapseltyps nach Anspruch 1, bei der die Oszillationseinheit (122) von einem Basis-Resonanzschema ist.

3. Medizinische Vorrichtung eines Kapseltyps nach Anspruch 1, bei der
die Oszillationseinheit einen PLL (Phasenregelkreis) aufweist und auch einer FSK, Frequenzumtastsignalisierungs, Modulationsfunktion dient.

4. Medizinische Vorrichtung eines Kapseltyps nach Anspruch 1, bei der
die Oszillationseinheit (411) von einem Kollektor-Resonanzschema ist.

5. Medizinische Vorrichtung eines Kapseltyps nach Anspruch 4, bei der
eine zwischen Kollektoranschlüssen der Oszillationseinheit (411) angeordnete Oszillationsinduktivität dazu vorgesehen ist, auch als eine Antenne zum Erzeugen eines elektromagnetischen Felds zu dienen, und in der drahtlosen Kommunikationsvorrichtung (41) enthalten ist.

## Revendications

1. Dispositif médical de type capsule destiné à être introduit dans un intérieur d'un corps vivant afin d'observer l'intérieur du corps vivant, comprenant :
un moyen de capture d'image (11) pour capturer une image de l'intérieur du corps vivant ;
un moyen de communication sans fil (12) pour transmettre des données d'image de l'image capturée par le moyen de capture d'image (11) jusqu'à un extérieur du corps vivant par l'intermédiaire d'une communication sans fil ;
un premier moyen régulateur de tension (13) pour réguler une tension délivrée en sortie d'un moyen d'alimentation électrique (16), et pour amener la tension jusqu'au moyen de capture d'image (11) ; et
un deuxième moyen régulateur de tension (14) pour réguler la tension délivrée en sortie du moyen d'alimentation électrique (16), et pour amener la tension qui est stable jusqu'au moyen de communication sans fil (12), dans lequel
une valeur de la tension délivrée par le deuxième moyen régulateur de tension (14) est plus petite qu'une valeur de la tension délivrée par le premier moyen régulateur de tension (13), et
une unité oscillante (122) comprise par le moyen de communication sans fil (12) a une configuration différentielle.

2. Dispositif médical de type capsule selon la revendication 1, dans lequel
l'unité oscillante (122) est d'une configuration à base résonante.

3. Dispositif médical de type capsule selon la revendication 1, dans lequel
l'unité oscillante a une PLL (boucle à verrouillage de phase), et sert également à une fonction de modulation FSK, par déplacement de fréquence.

4. Dispositif médical de type capsule selon la revendication 1, dans lequel
l'unité oscillante (411) est d'une configuration à collecteur résonant.

5. Dispositif médical de type capsule selon la revendication 4, dans lequel
un inducteur d'oscillation (65) agencé entre des bornes de collecteur de l'unité oscillante (411) est réalisé de façon à servir également comme une antenne pour générer un champ électromagnétique, et est compris par le dispositif de communication sans fil (41).
